# EUROPEAN PATENT APPLICATION

(11) **EP 2 989 898 A1**
(43) Date of publication of application: **02.03.2016**
(21) Application number: 15181630.3
(22) Date of filing: 19.08.2015
(51) Int. Cl.: A23G 3/34, A23G 3/02, A23G 3/20, A23G 3/36, A23G 3/42, A23G 3/54, A23G 4/04, A23G 4/10, A23G 4/20, A23G 7/00, A61K 9/00, A61K 9/68

(54) **PROCESS FOR PRODUCING MEDICATED FILLED CANDIES**

(30) Priority: 29.08.2014 IT BG20140038
(71) Applicant: Executive S.r.l., 24040 Pagazzano (BG) (IT)
(72) Inventor: RIZZI, Rodolfo, 24047 TREVIGLIO (BG) (IT); GAVIOLI, Maurizio, 20062 CASSANO D'ADDA (MI) (IT)
(74) Representative: Gatti, Enrico

(57) **Abstract**

A process for the production of filled medicinal candies comprising the steps of: preparing a mixture for a syrup based on a sweetener element with at least one active principle at a temperature below 70°C; preparing a mixture for candy; forming a casing of a candy, with said mixture for candy, by means of a rolling machine; send said casing to a rope sizer; inserting said mixture for a syrup in said casing of a candy to form a rope formed by said mixture for candy, filled of said mixture for a syrup; measuring the amount supplied of said mixture for a syrup by means of a mass flowmeter; setting the amount of said mixture for a syrup, by means of a delivery pump, according to measurements performed by said mass flowmeter; controlling the size of the rope; adjusting the speed of said rope sizer according to the sizes of said rope.

## Description

The present invention relates to a process for the production of filled candies, in particular filled medicinal candies.

Such a system and process is applicable also for the production of filled medicinal chewing gum.

The term medicinal candy means a candy filled with one or more active principles. The active principle is the component of medicines to which result its curative action, the medicine itself, that is indicates a substance that has a certain biological activity.

Are known medicinal candies that contain active principles that develop healing properties.

These active principles are incorporated in the candy dough, whose production involves mixing them with sugar and other ingredients to prepare the candy.

To carry out the mixing of the active principle with candy the process involves heating the whole to a temperature greater than 100°C and may even reach 150°.

At these temperatures it is possible that a portion and in any case not specified quantity, of the active principle might evaporate.

Then at the end of production cycle, it is uncertain what quantity of active principles remaining in the candy and even a constant quantity cannot be assured during production cycle.

The object of the present invention is to provide a process for the production of medicinal candies that can ensure a predefined quantity of active principles in the candy.

Another object is to ensure constancy of the quantity of active principle inserted.

In accordance with the present invention, these aims and others are achieved by a process for the production of filled medicinal candy comprising the steps of: preparing a mixture for a syrup based on an sweetener element with at least one active principle at a temperature below at 70°C; preparing a mixture for candy; forming a casing of a candy, with said mixture for candy, by means of a rolling machine; send said casing to a rope sizer; inserting said mixture for a syrup in said casing of a candy to form a rope formed by said mixture for candy, filled of said mixture for a syrup; measuring the amount supplied of said mixture for a syrup by means of a mass flowmeter; setting the amount of said mixture for a syrup, by means of a delivery pump, according to measurements performed by said mass flowmeter; controlling the size of the rope; adjusting the speed of said rope sizer according to the sizes of said rope.

Further characteristics of the invention are described in the dependent claims.

The advantages of this solution compared to the solutions of the prior art are various.

With the present process is realized a syrup, containing at least one active principle, at a temperature of 60°C reducing if not eliminating the evaporation of the active principle.

This syrup is then placed inside a candy to form a filled candy.

In this way, not mixing the candy with the active principle, and therefore not bringing it to high temperatures, it does not have any evaporation of the active principle.

With the predisposed control systems is possible to obtain a very precise control of the content of the syrup in the candy, and then a stable control in time of the content of active principle.

The characteristics and advantages of the present invention will become apparent from the following detailed description of an embodiment thereof, illustrated by way of non-limiting example in the accompanying drawings, in which:

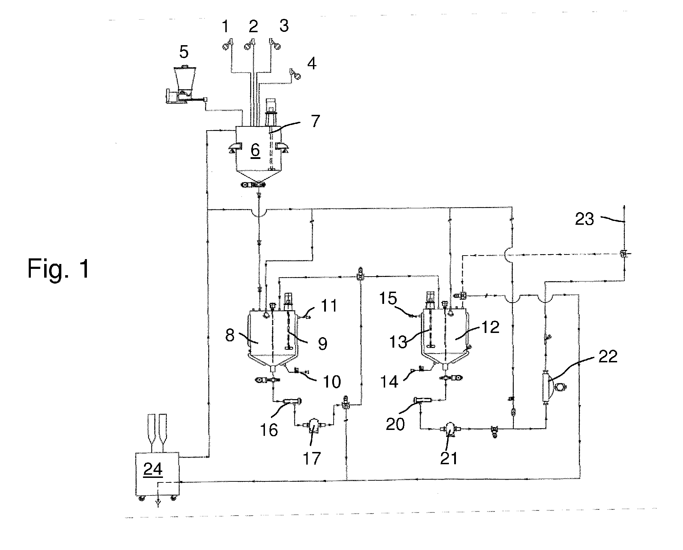

Figure 1 shows schematically a diagram of the production line of a syrup containing active principles, according to the present invention;

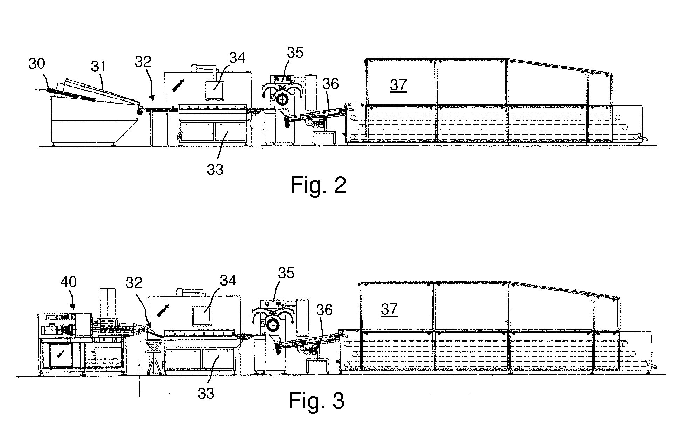

Figure 2 shows schematically a plant for the production of filled medicinal candies, in accordance with the present invention;
Figure 3 schematically shows a plant for the production of filled medicinal chewing gum, in accordance with the present invention;

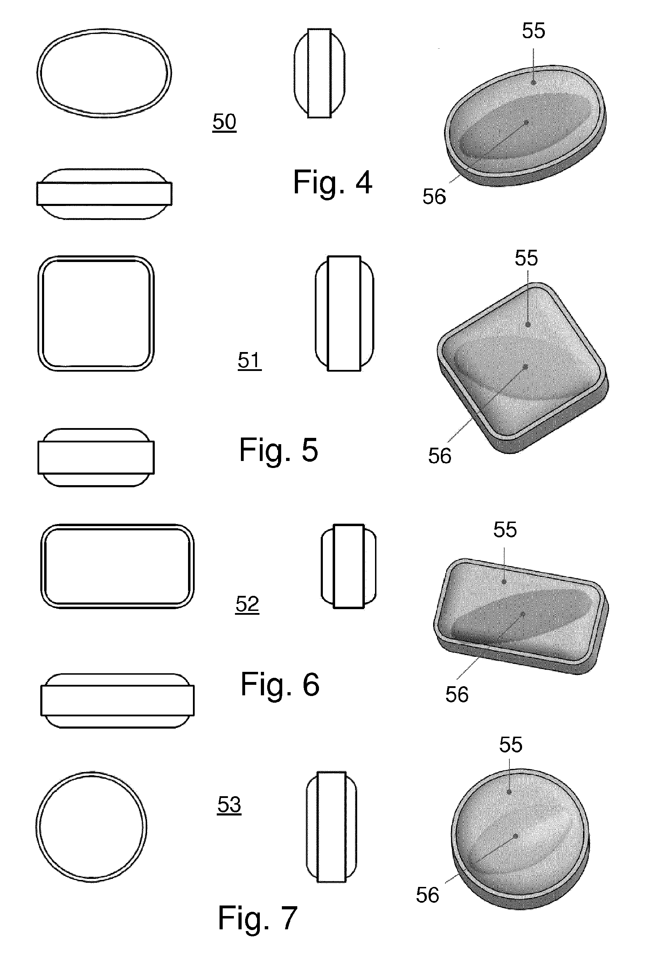

Figures 4-7 show various forms of filled medicinal candy according to the present invention.

The plant comprises a series of machines for the preparation of a syrup with one or more active principles which forms the filling of a candy.

The syrup has a composition of water, glucose, sugar, flavorings and active principles and is prepared at a temperature of 60°C, and in any case below 70°C.

As an alternative, for sugar-free candy, the syrup has a composition of isomalt, maltitol, flavorings and active principle and is prepared at a temperature of 60°C, and anyway below 70°C.

The quantity of the ingredients listed above is variable according to the needs and is of the type normally used for the preparation of similar mixtures.

The sugar (sucrose), which is obtained from the sugar cane or sugar beet, has its melting point is around 185°C, isomalt around 180°C and maltitol at around 150°C.

The preparation of the syrup, according to the present invention, takes place advantageously at a temperature much lower than the melting temperature and precisely at about 60°C where the sucrose (or other equivalent elements) soften (become viscous) and allow their mixing with the other ingredients and in particular with the active principles. The various components are weighed with electronic load cells, and then introduced individually by the inputs 1-5 in a first cylindrical container 6, water heated, equipped with a mixer 7 at high speed, adapted to obtain the desired solution, after various components and active principles have been dispersed in it. The active principles can be of liquid, granules or powder. The mixer 7 allows obtaining a product having a homogeneous structure of the molecules, thereby creating a syrup totally stable, complete with all the ingredients and ready for further processing.

Once reached the set temperature, not higher than 70°C, the syrup is discharged into a second stainless steel tank 8, with a double bottom heated with hot water, and also equipped with stirrer 9 to allow a storage phase. Where it is indicated with 10 the hot water inlet and with 11 is indicated the hot water outlet.

The final concentration is 80% of the dry substance.

It then proceeds to carry out any laboratory tests and take action to correct or confirm the suitability of the syrup solution. Obtained the suitability it will proceed to send the syrup in a third storage tank 12, with double bottom heated with hot water, and comprising a mixer 13. With 14 is indicated the hot water inlet and with 15 is indicated the hot water outlet.

The flow of syrup between the tank 8 and the tank 12 passes through a (optional) filter 16 (80 Mesh), and a pump 17.

The syrup output from the third storage tank 12 passes through a filter 20 (80 Mesh) (optional), and a pump 21.

The delivery pump 21 is self-regulated by a mass flowmeter 22 (which delivers in kg/h the amount of product passing through it), which is placed in succession to it, that control and set the amount of syrup that passes through it, towards the subsequent step of processing. The syrup is then sent to the outlet 23 of the production line.

The production line further includes piping, valves, and a container 24 for cleaning the plant components locally (CIP) without dismantling and without opening the system.

The candy casing is prepared in a known way with a composition of sugar, glucose, water, flavoring and colorants. For chewing gum, the composition contains a gum base, sweeteners, waxes, flavoring and colorants.

The production line for the ingredients includes a series of machines suitable to obtain a continuous production, a control system for the filling (with active principles) and a control system of the quantity of the filling solution containing the active principle with a tolerance of between 95% and 105%.

The syrup coming from the outlet 23 is conveyed to a Teflon® coated stainless steel tube 30 for transport of the syrup.

A roller 31 give to the candy casing the shape of a sugar rope that will contain the filling.

A first check of the size and weight of the rope takes place, at position 32, where the rope is fed to the successive crosspiece, and the speed of the successive rope sizer 33 is adjusted (and therefore of the production line) so to keep constant the diameter, which is an indispensable condition in order to obtain a rope which is as constant as possible when leaving the rope sizer, before the final calibration made on the former.

A rope sizer 33 follows, consisting of multiple calibration stations, which allows realizing a rope having a predetermined diameter and a determined filling quantity.

The rope is tridimensionally controlled by cameras 34, considering both the horizontal length and the vertical length, for the exact calculation of the diameter of the rope. This image is used for the correct calculation and for weight control. The final weight designed for each product, is in fact, directly related to the volume of the cylinder cut off from the die ring. The cutting pitch is fixed and known while the diameter of the cylinder and the specific weight of the product are the two variables that determine the final weight. Assumed that the specific weight is also constant for a given product, set in the recipe, the only variable at this point is the diameter of the rope. The instantaneous micrometric measure of the two horizontal and vertical lengths give a sufficiently precise information of the rope, enabling for calculating the volume and then, by means of the specific weight, the weight of the molded piece.

The cameras 34 have a hybrid technology, with integration of image and color with high-speed readers, for which in the case of production of hard and transparent candies and use of the filling of a color different from the candy (for instance a white or yellow transparent candy and blue or green filling) is also checked the actual presence and consistency of the filling, while, in the case of production of filled chewing-gum, since this is not transparent, the system is used to check for any leakage of the filling in the rope, being possible to discriminate two different colors.

Accurate control of the rope allows remarkable accuracy the ingredient quantities in the finished candy, therefore allows accurate control of the active principles contained therein.

Thereafter, the filled candies with syrup are printed in a forming equipment 35. This step provides for the closure of the filling inside the rope, and then the cutting of the finished candy.

The speed of the rope, which can be detectable both by the calibrator disc and by the die rotation speed, establishes the flow rate in kg/hour of the line. This data is processed and used to adjust the speed of the rope sizer 33. The quantity of filling is measured by the mass flowmeter 22, which adjust the flow rate of the pump 21 by means of an appropriate reaction.

An automatic mechanism 36, discard nonstandard product, acts on the belt at the forming equipment outlet, to ensure that are present only molded parts conform to the data set in the worksheet (recipe) of production, on the cooling belt 37. In the case of non-compliant portions of rope, they are cut and discarded.

The shape of the final product can vary according to the market requirement and needs, in other words it can be oval, round, rectangular or square. The percentage of filling contained in each single candy is 15% up to 30% of the weight of the candy itself.

The cooling belt 37 cools and stabilizes the product to be ready for the next and final packaging step.

With the same system it is possible to obtain medicinal candies with chewing gum and chewy casing, consisting of gum base, sugar and glucose.

The working process follows the procedure indicated hereafter. Preparation of the syrup as described above.

For the chewing gum working procedure, as an alternative to the roller 31, an extruder 40 is used, which is suitable to form a rope (casing), inside which the syrup filling is introduced by means of a pump, in the desired quantities required by recipe.

The other previously described machines and/or steps are also used in this case.

For each type of medicinal candy, the pharmaceutical companies define the active principle to be used with any excipients.

As active principle, it is possible use any type such as those already used for sore throats, as dichlorophenylcarbinol, amylmetacresol, dequalinium chloride, flurbiprofen, and related excipients, colorants, flavorings etc.

It is possible to use also other active principle for other symptoms.

The filled medicinal candies, can have any shape but preferably (plan view) oval 50, squared 51, rectangular 52 or circular 53, with a density of about 1.4 kg/dm^3, a volume between 2000 and 3000 mm^3 and a weight between 3 and 4 g.

With the numerical reference 55 is indicated the external casing of the candy and with the reference number 56 is indicated the syrup filling containing the active principles.

## Claims

1. A process for the production of filled medicinal candies comprising the steps of: preparing a mixture for a syrup based on a sweetener element with at least one active principle at a temperature below 70°C; preparing a mixture for candy; forming a casing of a candy, with said mixture for candy, by means of a rolling machine; send said casing to a rope sizer; inserting said mixture for a syrup in said casing of a candy to form a rope formed by said mixture for candy, filled of said mixture for a syrup; measuring the amount supplied of said mixture for a syrup by means of a mass flowmeter; setting the amount of said mixture for a syrup, by means of a delivery pump, according to measurements performed by said mass flowmeter; controlling the size of the rope; adjusting the speed of said rope sizer according to the sizes of said rope.

2. A process according to claim 1 **characterized in that** the phase of preparing said mixture for a syrup comprises the step of mixing water, glucose, sugar, aromas.

3. A process according to claim 1 **characterized in that** the step of preparing said mixture for candy comprises the step of mixing sugar, glucose, water, flavoring and colorants.

4. A process according to claim 1 **characterized in that** said filled medicinal candies have a density of about 1.4 kg/dm^3, a volume between 2000 and 3000 mm^3 and a weight between 3 and 4 g.

5. A process according to claim 1 **characterized in that** the step of preparing a mixture for a syrup comprises the phase of inserting said mixture for a syrup in a first cylindrical container (6) heated by water, and mixing said mixture for a syrup with a mixer (7) at high speed.

6. A process according to claim 1 **characterized in that** the phase of controlling the size of said rope comprises the use of cameras (34).

7. A process according to claim 6, **characterized in that** the phase of controlling the size of said rope comprises the phases of obtaining, by said cameras (34), a horizontal length and a vertical length of said rope.

8. A process according to claim 6, **characterized in that** the step of controlling the size of said rope comprises the phase of calculating the diameter of said rope.

9. A process according to claim 6, **characterized in that** the phase of controlling the size of said rope comprises the phase of calculating the weight based on the specific weight of said rope.

10. A process according to claim 6, **characterized in that** the step of controlling the size of said rope comprises the phase of controlling the actual presence and consistency of the filling in the case of hard and transparent candies and use of the filling of a color different from the candy.
